# EUROPEAN PATENT SPECIFICATION

(11) **EP 1 842 496 B1**
(45) Date of publication and mention of the grant of the patent: **11.11.2009**
(21) Application number: 07251402.9
(22) Date of filing: 30.03.2007
(51) Int. Cl.: A61B 17/128, A61F 5/00, A61B 17/30

(54) **Devices for compliant gastroplasty**
Vorrichtungen für anpassbare Gastroplastie
Dispositifs de gastroplastie adaptable

(30) Priority: 31.03.2006 US 278259
(43) Date of publication of application: 10.10.2007
(73) Proprietor: ETHICON ENDO-SURGERY, INC., Cincinnati, Ohio 45242 (US)
(72) Inventor: Ortiz, Mark S., Milford, Ohio 45150 (US)
(74) Representative: Tunstall, Christopher Stephen

(56) References cited:
- WO-A-99/60931
- WO-A-2005/034729
- WO-A-2005/037152
- US-A1- 2005 149 200

## Description

### FIELD OF THE INVENTION

The present application is directed to devices for forming a gastroplasty, and in particular to such devices that can effect a gastroplasty having a compliant nature.

### BACKGROUND OF THE INVENTION

Severe obesity is a major health risk that can decrease life expectancy and give rise to a number of other associated ailments including the onset of cardiovascular disease, hypertension, diabetes and severe arthritis. A number of surgical procedures can be performed to aid in the treatment of obesity. One example is a gastric restriction, also known as a gastroplasty, in which one or more fasteners are inserted into gastric tissue to hold the tissue in a folded configuration that reduces the effective volume of a patient's stomach.

WO9960931 discusses a system including an implanted fastener for fastening layers of tissue. In one emhodiment, the fastener includes a proximal anchor member, and a distal anchor member, each being movable from a reduced profile position to a deployed position. The anchor members are mesh structures capable of moving to the deployed position by reducing the axial spacing between the opposite ends of the anchor members.

WO2005037152, which regarded as the closest prior art, discusses various methods and devices for retaining a medical implant within a body cavity. According to one aspect, at least a portion of a medical implant is positioned within a body cavity, and a wall of the body cavity is reshaped such that the re-shaped wall prevents migration of the medical implant out of the body cavity. The reshaped body wall may form a tissue pocket, tunnel, or other barrier against migration of the implant.

Due to the chronic, fluctuating forces acting upon the gastric walls of a patient and the constant movement of the stomach, gastric restrictions often have a limited lifetime. For example, large chronic forces (i.e., pressures) can act in the stomach due to any number of circumstances such as super-physiological events. Since current fasteners form a non-compliant coupling between the walls of the stomach, pressures that exceed the ability of the fasteners to maintain gastric tissue coupling may be relieved by unintentional separation of fasteners from the gastric walls. In such an instance, the gastric restriction needs to be surgically reapplied, which is troublesome for both the surgeon and the patient.

Accordingly, a need exists for forming gastric restrictions that are more robust to the cyclical forces acting on a patient's stomach. Furthermore, a need for devices that can create such gastric restrictions also exists.

### SUMMARY OF THE INVENTION

An exemplary embodiment is directed to a system for deploying one or more fasteners in gastric tissue to create a gastroplasty. The gastric restriction can be formed by arranging fasteners in a selected pattern. The system includes an insertion element having an end effector that can be endoscopically deployable (e.g., by a trans-oral route). One or more tissue positioning structures can be positioned on the end effector, along with one or more tissue penetrating probes. The tissue positioning structure can be embodied as one or more suction ports that are effective for adhering tissue to the structure. A trough can also be included with the tissue penetrating structure, one or more suction ports being optionally coupled to one or more walls of the trough. A tissue penetrating probe can be selectively deployable through a portion of the insertion element. For example, a probe can be advanced out of, and/or retracted from, an end effector using a variety of mechanisms, such as engaging a set of gear teeth on the probe with a rotatable wheel. In another example, the probe can be advanced into the trough through an opening. The probe can be configured to penetrate tissue (e.g., using a distal penetrating tip), and can also include a tissue stop for limiting probe penetration through tissue. A flexible elongate body can be included to help orient the probe. The probe can also be configured to deliver a magnetic fastener forming composition through one or more lumens to yield a magnetic fastener. For example, the probe can include two or more lumens and a static mixing nozzle for delivering the magnetic fastener forming composition. Potential magnetic fastener forming compositions include solidifiable gel mixtures that can include magnetic particles that tend to align their magnetic dipoles. Multiple probes and multiple troughs can be utilized with the end effector to deliver the fasteners. For example, two tissue penetrating probes can be located on opposing walls of a trough to penetrate adjacent tissue, or separate troughs can be configured to adhere gastric tissue on the anterior side and on the posterior side of the stomach.

Another exemplary embodiment is directed to a gastric restriction fastener system that includes multiple magnetic fasteners. Fasteners can be shaped with a narrow intermediate portion configured to extend through gastric tissue, and widened portions on the ends for placement adjacent to a gastric tissue surface. Fasteners can be adapted to be embedded in gastric tissue, and oriented in a desired pattern to effect a gastroplasty of a patient's stomach. As well, each of the fasteners can be configured to magnetically adhere to one or more other fasteners. Coupled fasteners can also reversibly decouple in response to a separation pressure in excess of a predetermined threshold value, e.g., a value between about 6.9 and 20.7 K.lo pascal (1 pound per square inch and about 3 pounds per square inch).

A method of creating a compliant gastroplasty is presented. Multiple fasteners, which each reversibly couple to one or more other fasteners, can be inserted into a gastric wall in a selected pattern to form a restricted volume within a stomach. For example, one or more lines of fasteners can be inserted, and such lines can be located on the anterior and posterior walls of the stomach to effect the gastric restriction. Types of fasteners include magnetic fasteners that tend to magnetically couple to another magnetic fastener. Fasteners can be formed from a solidifying composition that is inserted into the gastric wall. When a magnetic fastener is formed using a solidifying composition, the composition can include a dispersion of magnetic particulates. Fasteners can be configured to tend to decouple when the restricted volume of the stomach is subjected to a separation pressure in excess of a predetermined value, such as about 6.9 KPa (1 pound per square inch). The method can be performed endoscopically (e.g., trans-orally) to utilize a minimally-invasive surgical technique.

### BRIEF DESCRIPTION OF THE DRAWINGS

The invention will be more fully understood from the following detailed description taken in conjunction with the accompanying drawings, in which:
FIG. 1 presents an anterior view of a stomach having a plurality of fasteners attached thereto consistent with an embodiment of the invention;
FIG. 2 presents a cross-sectional view of a stomach having reversibly coupled fasteners attached to an anterior wall and a posterior wall of the stomach;
FIG. 3A presents an anterior view of a stomach with a cutaway portion showing an end effector of a fastener deployment system within the stomach cavity;
FIG. 3B a close up view of the cutaway portion of the stomach shown in FIG. 3A;
FIG. 3C shows a perspective view of an end effector of a fastener deployment system having a layer of tissue oriented within a trough of the end effector;
FIG. 4 shows a perspective view of a tissue layer with a tissue penetrating probe piercing the tissue layer;
FIG. 5 shows a perspective view of a tissue penetrating probe; and
FIG. 6 shows a fastener embedded in a gastric wall.

### DETAILED DESCRIPTION OF THE INVENTION

Certain exemplary embodiments will now be described to provide an overall understanding of the principles, structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with features of other embodiments. Such modifications and variations are intended to be included within the scope of the present invention.

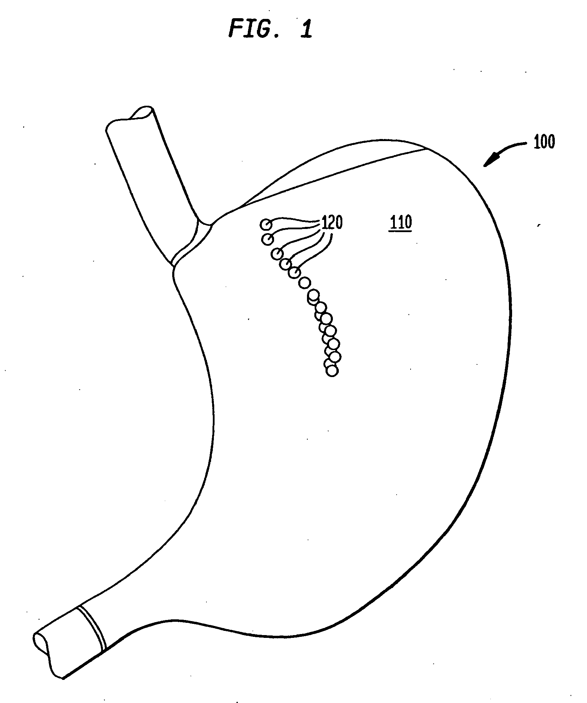

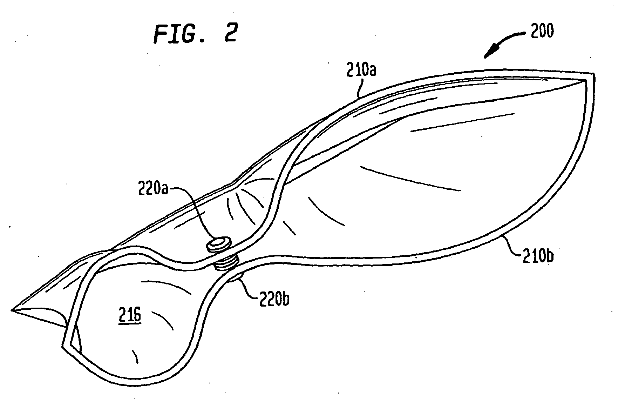

FIG. 1 presents an illustration of a gastric restriction fastener system coupled to an anterior side of a stomach 100, consistent with an exemplary embodiment. The system includes a plurality of fasteners that are embedded into gastric tissue and oriented in a desired pattern to effect a gastroplasty. For example, a set of fasteners 120 can be embedded into the anterior wall 110 of the stomach, as shown in FIG. 1, with a corresponding set of fasteners also embedded into the posterior wall (not shown). As depicted in the cross-sectional view of a stomach 200 in FIG. 2, the volume of the stomach 200 can be effectively restricted to a small volume 216 by the coupling of fasteners 220a, 220b that are each embedded in an opposed gastric wall 210a, 210b, respectively. In general, each of the fasteners 220a can be adapted to reversibly couple to at least one other fastener 220b. For example, fasteners can be in contact with one another during coupling, while sufficient tensile forces can cause the fasteners to separate. Upon sufficient relief from the tensile forces, the fasteners can recouple to contact each other again. Accordingly, fasteners 220a, 220b can be adapted to reversibly decouple in response to a separation pressure within the stomach 200 in excess of some predetermined value.

In use, reversibly coupled fasteners are typically coupled to form a gastric restriction. If some event causes the pressure in the stomach to exceed a predetermined pressure value, the reversibly coupled fasteners can decouple to effectively increase the volume of the stomach, and thereby relieve the excessive pressure without causing the fasteners to be torn or removed from the gastric wall. When the pressure in the stomach returns to a nominal level, shrinkage of the stomach can cause the anterior and posterior gastric walls to approach one another. Fasteners that are brought back into proximity to one another by the contracting stomach walls can recouple, thereby reforming the gastric restriction. In general, if a set of fasteners are within the anterior wall of the stomach (as shown in FIG. 1 for example), with a corresponding set of fasteners embedded within the corresponding posterior wall, each fastener in the anterior wall need not attach to an exact corresponding fastener in the posterior wall. Indeed, after decoupling and recoupling to reform a gastric restriction, some fasteners may not be coupled to another fastener, or multiple fasteners may be coupled to one fastener. The reversible nature of fastener coupling can allow a gastric restriction to form, break apart, and reform, i.e., the gastric restriction can be self-correcting. As such, the gastric restriction fastener system provides a compliant gastroplasty that can withstand super-physiological events.

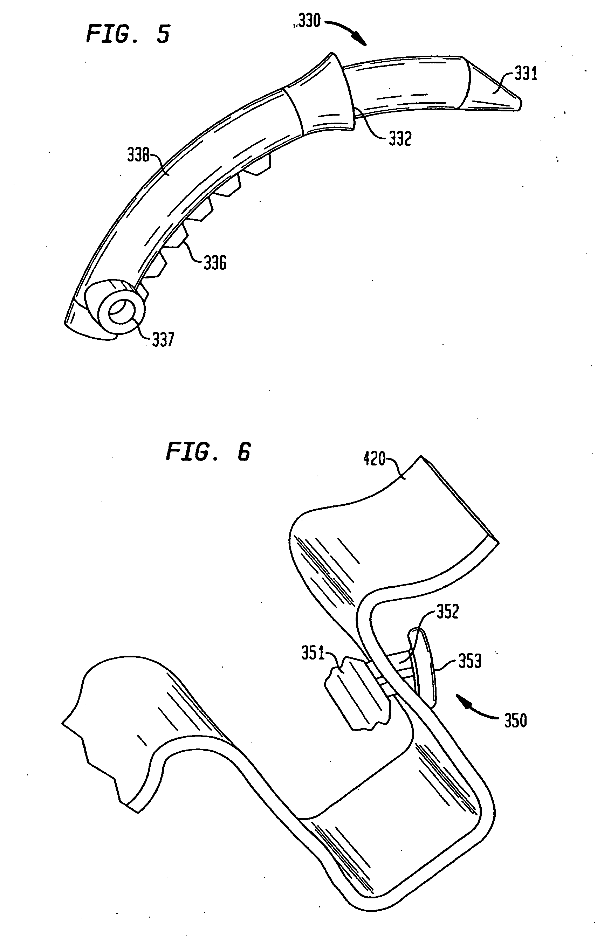

Fasteners utilized with a gastric restriction fastener system can be configured in a variety of shapes and sizes to reversibly couple and decouple in a manner consistent with a compliant gastroplasty. For example, the fastener can be configured to penetrate gastric tissue, with a narrow intermediate portion 352 extending through the tissue and wider portions 351, 353 located on each end of the intermediate portion 352 that are adjacent to a tissue surface 420 as exemplified by the fastener 350 depicted in FIG. 6. As mentioned earlier, fasteners can be arranged in a desired pattern to form the gastric restriction. The restriction can proceed along a path extending from near the cardiac orifice to the pylorus, or some intermediate path as depicted by the restriction shown in FIG. 1. Any number of fasteners can be used to create the reversible gastric restriction. For example, pairs of coupled fasteners can be located about 2 millimeters to about 8 millimeters apart, or preferably about 4 millimeters to about 5 millimeters apart. As well, one or more linear or non-linear rows of fasteners can be used to form the restriction.

Fasteners can also be constructed from a variety of materials. In one embodiment, each fastener can be embodied as a magnetic fastener that can magnetically adhere to one or more other fasteners. As such, the magnetic fastener can also be configured to reversibly couple and decouple. In particular, a magnetic fastener can be formed from a fastener forming composition (e.g., a gel composition) that solidifies into a final fastener shape. The fastener forming composition can include magnetic particulates that tend to align their magnetic dipoles. One example of a fastener forming composition is a gel composition of polymer solution including polyvinyl alcohol combined with nanosized iron oxide particles (e.g., γ-Fe₂O₃, ~ 7 nm), as described by Chattergee et al. (BioMagnetic Research and Technology 2004, 2:2). Upon mixing, the composition quickly forms a gel that dries and solidifies. As such, the gel can quickly set into a desired shape, such as that previously described. Those skilled in the art will readily appreciate that a number of other types of compositions can be utilized to form a compliant gastroplasty.

As previously mentioned, reversible decoupling of fasteners can occur when the pressure within the stomach exceeds a predetermined value. For example the predetermined value can be greater than about 6.9 KPa (1 pound per square inch), or in the range of about 6.9 KPa to about 20.7 KPa (1 pound per square inch to about 3 pounds per square inch), or the predetermined value can be about 3.8 KPa (2 pounds per square inch). The strength of the magnetic attraction between fasteners can be regulated to obtain the desired predetermined value at which a gastric restriction would be released to relieve internal stomach pressure.

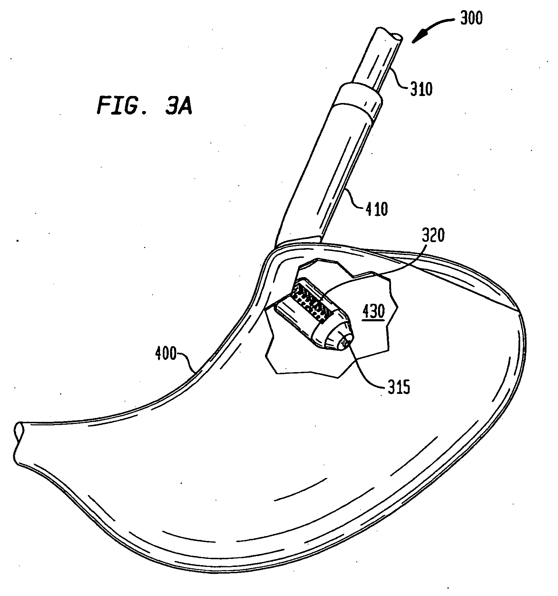

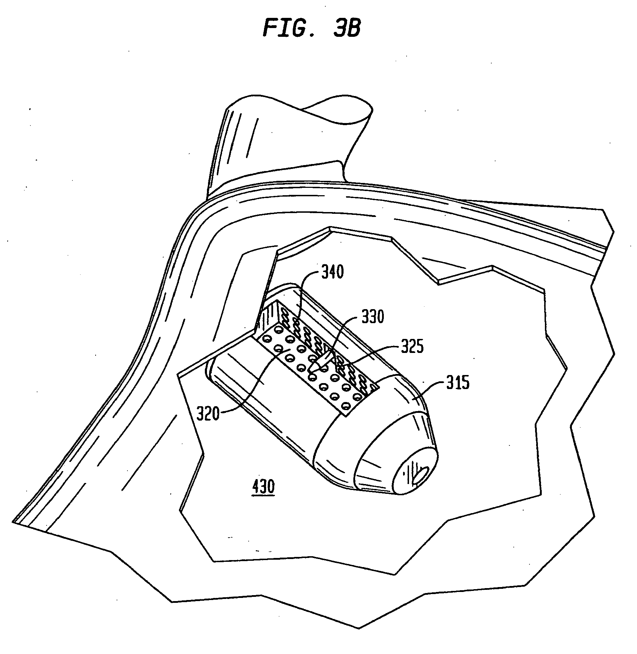

Another embodiment is directed to a system for deploying fasteners in gastric tissue to effect a gastroplasty. Such a system is generally represented by the device 300 depicted in FIGS. 3A-3C. The device 300 can include an endoscopically deployable insertion element having an end effector 315. The end effector 315 can be coupled to a shaft 310 of the insertion element by a coupling 360 that can be configured to advance and/or angle the end effector 315. The end effector can include one or more tissue positioning structures, and can also be associated with one or more tissue penetrating probes that can be selectively deployable through a portion of the insertion element. For example, as depicted in FIG. 3B, the probe 330 can be deployed through a slot 325 (or other opening) and into the trough 320 of an end effector 315. A probe can also include one or more lumens for delivering a fastener forming composition. One skilled in the art will appreciate that the device 300, including shaft 310 and end effector 315, is of a type of construction that is suitable for endoscopic delivery, such as trans-oral delivery. For example, shaft 310 can be an elongate member that is of sufficient flexibility, and/or selectively flexible, to traverse a tortuous path within a lumen of the body.

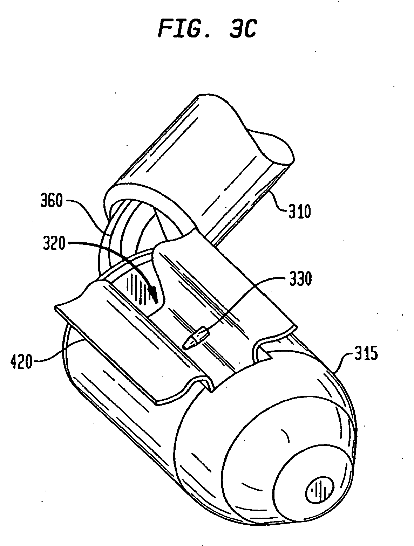

In general, tissue positioning structures can orient and/or position tissue (e.g., the gastric wall) to facilitate insertion of a fastener element. For the exemplary embodiment shown in FIGS. 3A-3C, the end effector 315 includes a trough 320 having one or more suction ports 340 disposed in the inner wall. A suction port can be configured to adhere tissue thereto, such as depicted in FIG. 3C where the suction ports 340 draw the gastric tissue 420 into the trough 320. A trough configuration can be advantageous by providing isolation for a tissue penetrating probe that pierces the gastric tissue, and hindering potential collateral damage that can be associated with tissue piercing by the probe. As depicted by the anterior cutaway view of the stomach shown in FIG. 3A, the end effector 315 can be configured with a trough 320 that can be positioned to adhere gastric tissue 400 from the anterior side of the stomach. The end effector 315 can also include an additional trough (not shown) opposite the illustrated trough 320 for adhering gastric tissue from the posterior side of the stomach. In such a configuration, an end effector can apply a fastener to each side of the stomach (as shown in FIG. 2) for forming the gastric restriction. Though FIGS. 3A-3C show an exemplary embodiment of an end effector with a tissue positioning structure, those skilled in the art will readily appreciate that a variety of other tissue positioning structure configurations can be effectively utilized with a fastener deployment system. For example, the use of a trough is not required as part of tissue positioning structure. One or more suction ports can be utilized without a trough for adhering tissue. Furthermore, other types of tissue positioning structures besides a suction port can be used to orient tissue in a desired configuration for penetration by a tissue penetrating probe (e.g., tissue graspers, clamps, and other tissue manipulating devices). All of these variations are within the scope of the present application.

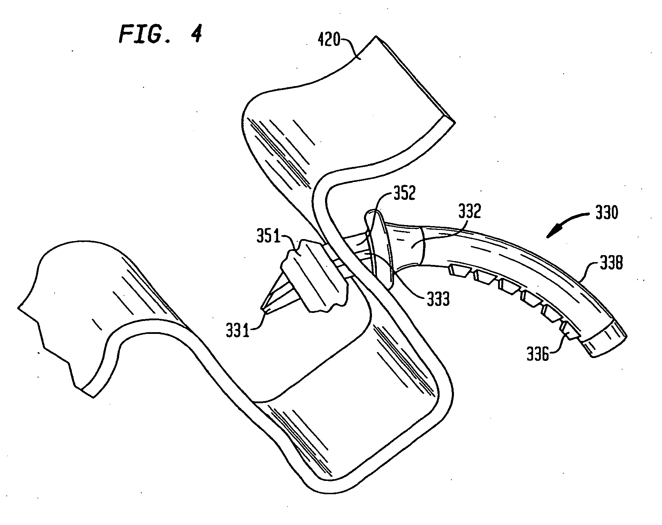

Tissue penetrating probes used with an insertion element can be configured in variety of manners to allow tissue penetration and probe manipulation (e.g., advancement out of, or retraction into, an end effector). An exemplary probe 330 is depicted in FIG. 5. A probe 330 can include a distal penetrating tip 331 for tissue penetration. The probe can also include a tissue stop configured to limit penetration of the probe into the tissue, such as the inverted flare structure 332 shown in FIGS. 4 and 5. A flexible elongate body 338 can be used to aid orientation and manipulation of the probe 330. A plurality of gear teeth 336 can be distributed along a portion of the probe 330 and configured to engage a rotatable gear wheel (not shown), which can be located within the insertion element. By rotating the gear wheel in a desired direction, the probe can be advanced or retracted. Those skilled in the art will readily appreciate that probes can be configured in a variety of other configurations beyond what is described herein, and can be utilized with tissue positioning structures in a various arrangements. For example, two tissue penetrating probes can be deployed through opposing inner walls of a trough and into adjacent tissue to form fasteners therein, either serially or simultaneously. Indeed, such arrangements, among others, can deliver a plurality of fasteners in a selected pattern arrangement to aid in forming a gastric restriction.

As previously discussed, a probe 330 can include a lumen 333 (shown in FIG. 4) for delivering a magnetic fastener forming composition as described herein (e.g., a solidifiable gel mixture), the composition entering the lumen through a port 337 as depicted in FIG. 5. Since some fastener forming compositions solidify quickly under particular conditions, it can be advantageous to configure a probe to compartmentalize stable components of the composition and combine the components prior to distributing the composition to yield a fastener. For example, a probe can contain two or more lumens and a static-mixing nozzle, with the plurality of lumens holding separate components of the fastener forming composition. Upon moving the components of each lumen into the nozzle, the components are mixed to form the fastener forming composition. The components, or the entire premixed fastener forming composition, can be stored within the probe 330 (e.g., within the one or more lumens or in one or more reservoirs in the probe). Alternatively, the components or entire composition can be stored elsewhere within the fastener deployment system. For example, the materials can be stored in one or more separate reservoirs, and can be subsequently delivered to the probe. Such reservoirs can be stored within a portion of the insertion element, or can be completely separated from the insertion element, but placed in fluid communication with the insertion element by flexible tubing or other portal.

In use, with reference to FIGS. 3A-3C, a fastener deployment system can be configured to be delivered trans-orally, through the esophagus 410 and into the cavity 430 of the stomach, as shown in the cutaway view of FIGS. 3A and 3B. Suction can be applied through the ports 340 to adhere gastric tissue 420 into the trough 320 of the end effector 315 depicted in FIG. 3C. A tissue penetrating probe 330 can be advanced through tissue as shown in FIG. 3C and the close-up perspective view of FIG. 4. The magnetic fastener forming composition can then be delivered through the probe 330 to form a fastener, such as the fastener 350 shown in FIG. 6. In general, the probe can be retracted or advanced as the fastener is formed, with the rate of composition release being controlled to aid in fastener shape formation. Upon composition solidification, a magnetic fastener is formed into the gastric wall. This process can repeated multiple times to form multiple fasteners. Accordingly, the composition is oriented to create a fastener with poles oriented to induce attraction with another fastener. Typically, magnetic particulates in a dispersion can orient themselves. However, a magnetic pole can be induced in the end effector, or other section of the insertion element, to cause fasteners in the posterior wall to attract fasteners in the anterior wall. Alternatively, if the end effector, or insertion element, is slightly magnetic, particles can orient in response to the magnetic field. After fastener formation, the fastener can be separated from the end effector using light insufflation, or other separation techniques. Upon formation of fasteners in a desired pattern, one or more fasteners can couple, for example as shown in FIG. 2, to form a gastric restriction. Coupling can be promoted by bringing the opposed fasteners into proximity, such as by the application of suction within the stomach cavity.

A method of creating a compliant gastroplasty is presented. The method can be performed endoscopically, such as in a trans-oral manner, with a plurality of fasteners being inserted into a gastric wall. The fasteners can be arranged in a predetermined pattern, such as in one or more lines extending in a manner to form a gastric restriction (e.g., one line in the anterior wall and a corresponding line in the posterior wall). One or more fasteners can be adapted to reversibly couple to at least one other fastener to form a restricted volume within a stomach. For example, the fasteners can be configured to decouple when the restricted volume is subjected to an expanding pressure in the stomach greater than about 6.9 KPa (1 pound per square inch). The fasteners are magnetic, having a tendency to magnetically couple to another fastener. Such magnetic fasteners can be formed using a fastener forming composition in a dumbbell-like shape, as described herein.

In another aspect, fastener deployment systems, including portions thereof, can be designed to be disposed of after a single use, or they can be designed to be used multiple times. In either case, however, the system can be reconditioned for reuse after at least one use. Reconditioning can include any combination of the steps of disassembly of the system, followed by cleaning or replacement of particular pieces, and subsequent reassembly. By way of example, the fastener deployment system shown in FIGS. 3A-3C and 5 can be reconditioned after the system has been used in a medical procedure. The device can be disassembled, and any number of the particular pieces (e.g., shaft 310, the end effector 315, the tissue penetrating probe 330 including any portions of the probe, etc.) can be selectively replaced or removed in any combination. For instance, the end effector can be replaced by a new end effector, while the remaining pieces of the insertion element are sterilized for reuse. Replacement of pieces can also include replacement of portions of particular elements, such as the replacement of a distal tip on a tissue penetrating probe. Upon cleaning and/or replacement of particular parts, the system can be reassembled for subsequent use either at a reconditioning facility, or by a surgical team immediately prior to a surgical procedure. Those skilled in the art will appreciate that reconditioning of a fastener deployment system can utilize a variety of techniques for disassembly, cleaning/replacement, and reassembly. Use of such techniques, and the resulting reconditioned system, are all within the scope of the present application.

Persons skilled in the art will understand that the devices specifically described herein and illustrated in the accompanying drawings are non-limiting exemplary embodiments. The features illustrated or described in connection with one exemplary embodiment may be combined with the features of other embodiments. Such modifications and variations are intended to be included within the scope of the present invention. As well, one skilled in the art will appreciate further features and advantages of the invention based on the above-described embodiments. Accordingly, the invention is not to be limited by what has been particularly shown and described, except as indicated by the appended claims.

## Claims

1. A system for deploring fasteners (220a, 220b) in gastric tissue (210a, 210b) to effect a gastroplasty, comprising:
an endoscopically deployable insertion element having an end effector (315) with at least one tissue positioning structure formed thereon;
at least one tissue penetrating probe (330) associated with the end effector and selectively deployable through a portion of the insertion element;
**characterised in that;**
the system further comprises a magnetic fastener forming composition;
the at least one probe is configured to penetrate a layer of tissue and deliver the magnetic fastener forming composition through a lumen (333) of the at least one probe to yield a magnetic fastener that extends through opposed walls of the layer of gastric tissue; and
each of the fasteners is configured to magnetically adhere to at least one other fastener to form a gastric restriction and to reversibly decouple in response to a separation pressure in excess of a predetermined threshold valve.

2. The system of claim 1, wherein the at least one probe includes at least two lumens and a static-mixing nozzle for delivering the fastener forming composition.

3. The system of claim 1, wherein the magnetic fastener forming composition comprises a solidifiable gel mixture.

4. The system of claim 3, wherein the solidifiable gel mixture comprise magnetic particles tending to have aligned magnetic dipoles.

5. The system of claim 1, wherein the at least one probe includes a distal penetrating tip (331) and a tissue stop located proximal to the tip for limiting penetration of the at least one probe through the tissue.

6. The system of claim 1, wherein the at least one probe is configured to he advanced out of the end effector and retracted into the end effector.

7. The system of claim 1, wherein the at least one probe includes a flexible elongate body (338).

8. The system of claim 1, wherein a plurality of gear teeth (336) are distributed along a section of the at least one probe for engaging a rotatable gear wheel in the insertion element that advances and retracts the at least one probe.

9. The system of claim 1, wherein the at least one tissue positioning structure incudes at least one suction port (330) effective to adhere tissue thereto.

## Patentansprüche

1. System zur Ablage von Befestigungselementen (220a, 220b) in Magengewebe (210a, 210b) zur Durchführung einer Gastroplastie, umfassend:
ein endoskopisch ablegbares Einführelement mit einem Endeffektor (315) mit mindestens einer daran ausgebildeten Gewebepositionierstruktur;
mindestens eine gewebedurchdringende Sonde (330), die mit dem Endeffektor verbunden und durch einen Abschnitt des Einführelements wahlweise ablegbar ist;
**dadurch gekennzeichnet, dass**
das System ferner eine ein magnetisches Befestigungselement bildende Zusammensetzung aufweist;
die mindestens eine Sonde zum Durchdringen einer Gewebeschicht und Zuführen der ein magnetisches Befestigungselement bildenden Zusammensetzung durch ein Lumen (333) der mindestens einen Sonde konfiguriert ist, um ein magnetisches Befestigungselement zu liefern, das sich durch gegenüberliegende Wände der Magengewebeschicht erstreckt; und jedes der Befestigungselemente zum magnetischen Anhaften an mindestens einem anderen Befestigungselement zur Bildung einer Magenbegrenzung und zum Entkoppeln in entgegengesetzter Richtung als Reaktion auf einen Ablösedruck oberhalb eines vorab festgelegten Schwellenwerts konfiguriert ist.

2. System nach Anspruch 1, **dadurch gekennzeichnet, dass** die mindestens eine Sonde mindestens zwei Lumen und eine statische Mischdüse zum Zuführen der ein Befestigungselement bildenden Zusammensetzung enthält.

3. System nach Anspruch 1, **dadurch gekennzeichnet, dass** die ein magnetisches Befestigungselement bildende Zusammensetzung ein verfestigbares Gelgemisch umfasst.

4. System nach Anspruch 3, **dadurch gekennzeichnet, dass** das verfestigbare Gelgemisch magnetische Partikel mit der Neigung zu ausgerichteten magnetischen Dipolen umfasst.

5. System nach Anspruch 1, **dadurch gekennzeichnet, dass** die mindestens eine Sonde eine distale Durchdringspitze (331) und einen Gewebeanschlag enthält, der sich proximal zur Spitze zum Begrenzen des Durchdringens der mindestens einen Sonde durch das Gewebe angeordnet ist.

6. System nach Anspruch 1, **dadurch gekennzeichnet, dass** die mindestens eine Sonde zum Herausbewegen aus dem Endeffektor und Hineinziehen in den Endeffektor konfiguriert ist.

7. System nach Anspruch 1, **dadurch gekennzeichnet, dass** die mindestens eine Sonde einen flexiblen länglichen Körper (338) enthält.

8. System nach Anspruch 1, **dadurch gekennzeichnet, dass** eine Vielzahl von Zahnrädern (336) entlang eines Abschnitts der mindestens einen Sonde für einen Eingriff mit einem drehbaren Zahnrad in dem Einführelement verteilt sind, das die mindestens eine Sonde vorbewegt und zurückzieht.

9. System nach Anspruch 1, **dadurch gekennzeichnet, dass** die mindestens eine Gewebepositionierstruktur mindestens eine Ansaugöffnung (330) zum Festhalten von Gewebe an selbiger enthält.

## Revendications

1. Système pour déployer des fixations (220a, 220b) dans le tissu gastrique (210a, 210b) afin d'effectuer une gastroplastie, comprenant :
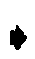 un élément d'insertion pouvant être déployé de manière endoscopique ayant un effecteur terminal (315) avec au moins une structure de positionnement de tissu formée sur celui-ci ;
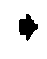 au moins une sonde de pénétration de tissu (330) associée à l'effecteur terminal et pouvant se déployer sélectivement à travers une partie de l'élément d'insertion ;
**caractérisé en ce que** :
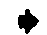 le système comprend en outre une composition de formation de fixation magnétique ;
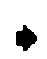 la au moins une sonde est configurée pour pénétrer dans une couche de tissu et pose la composition de formation de fixation magnétique à travers une lumière (333) de la au moins une sonde pour produire une fixation magnétique qui s'étend à travers les parois opposées de la couche de tissu gastrique ; et
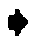 chacune des fixations est configurée pour adhérer magnétiquement à au moins une autre fixation afin de former une restriction gastrique et pour se découpler de manière réversible en réponse à une pression de séparation supérieure à une valeur de seuil prédéterminée.

2. Système selon la revendication 1, dans lequel la au moins une sonde comprend au moins deux lumières et une buse de mélange statique pour distribuer la composition de formation de fixation.

3. Système selon la revendication 1, dans lequel la composition de formation de fixation magnétique comprend un mélange de gel solidifiable.

4. Système selon la revendication 3, dans lequel le mélange de gel solidifiable comprend des particules magnétiques ayant tendance à avoir les dipôles magnétiques alignés.

5. Système selon la revendication 1, dans lequel la au moins une sonde comprend une pointe de pénétration distale (331) et un arrêt de tissu situé à proximité de la pointe afin de limiter la pénétration de la au moins une sonde à travers le tissu.

6. Système selon la revendication 1, dans lequel la au moins une sonde est configurée pour être avancée hors de l'effecteur terminal et rétractée dans l'effecteur terminal.

7. Système selon la revendication 1, dans lequel la au moins une sonde comprend un corps allongé flexible (338).

8. Système selon la revendication 1, dans lequel une pluralité de dents d'engrenage (336) sont réparties le long d'une section de la au moins une sonde pour mettre en prise une roue dentée rotative dans l'élément d'insertion qui fait avancer et rétracte la au moins une sonde.

9. Système selon la revendication 1, dans lequel la au moins une structure de positionnement de tissu comprend au moins un orifice d'aspiration (330) efficace pour y faire adhérer le tissu.
